# FASCICULE DE BREVET EUROPEEN

(11) **EP 1 963 249 B1**
(45) Date de publication et mention de la délivrance du brevet: **28.07.2010**
(21) Numéro de dépôt: 06819799.5
(22) Date de dépôt: 28.11.2006
(51) Int. Cl.: C07C 65/17, A61K 31/192, A61P 17/00, A61P 17/06, A61P 17/10

(54) **DERIVES BIPHENYLIQUES AGONISTES SELECTIFS DU RECEPTEUR RAR-GAMMA**
BIPHENYLDERIVATE ALS SELEKTIVE AGONISTEN VON GAMMA-RAR-REZEPTOREN
BIPHENYL DERIVATIVES AS SELECTIVE AGONISTS OF GAMMA RAR RECEPTORS

(30) Priorité: 15.12.2005 FR 0512762
(43) Date de publication de la demande: 03.09.2008
(73) Titulaire: Galderma Research & Development, 06410 Biot (FR)
(72) Inventeur: BIADATTI, Thibaud, F-06650 Opio (FR); THOREAU, Etienne, F-06460 Saint Vallier De Thiey (FR)
(74) Mandataire: Allab, Myriam
(86) Numéro de dépôt international: PCT/EP2006/068975
(87) Numéro de publication internationale: WO 2007/068579

(56) Documents cités:
- WO-A-99/10308
- WO-A-2005/056516
- DAWSON M I ET AL: "4-[3-(5,6,7,8-Tetrahydro-5,5,8,8-tetramet hyl-2-naphthalenyl)phenyl]be nzoic acid and heterocyclic-bridged analogues are novel retinoic acid receptor subtype and retinoid X receptor alpha agonists" BIOORGANIC & MEDICINAL CHEMISTRY LETTERS, OXFORD, GB, vol. 10, no. 12, juin 2000 (2000-06), pages 1311-1313, XP004206994 ISSN: 0960-894X

## Description

La présente invention se rapporte à l'utilisation en thérapie, notamment dans le domaine de la dermatologie, de composés biphényliques substitués par un radical aromatique à activité sélective pour le sous-type gamma de la famille des récepteurs RAR.

Une famille de composés biphényliques a été décrite dans la demande de brevet WO 99/10308. Ces composés sont décrits comme ayant une application dans le traitement topique et systémique des affections dermatologiques liées à un désordre de la kératinisation et des affections ophtalmologiques, notamment.

L'activité de ces composés a notamment été mise en évidence par des tests de différenciation des cellules F9 de tératocarcinome embryonnaire de la souris et des tests de différenciation des kératinocytes chez l'homme.

En revanche, ce document ne fait nullement état d'une éventuelle activité spécifique des composés vis-à-vis du sous-type gamma des récepteurs RAR.

Or, le sous type gamma de la famille des récepteurs RAR est largement majoritaire dans l'épiderme où il représente environ 90% du total des récepteurs (« Retinoic acid receptors and binding proteins in human skin », Elder JT, Astrom A, Pettersson U, Tavakkol A, Krust A, Kastner P, Chambon P, Voorhees JJ : J Invest Dermatol. 1992;98 (6 Suppl): 36S-41S ; ou : "Retinoic acid receptor expression in human skin keratinocytes and dermal fibroblasts in vitro", Redfern CP, Todd C. J Cell Sci. 1992;102 (Pt 1):113-21) et c'est bien l'interaction avec ce récepteur RAR gamma qui est responsable de l'efficacité des rétinoïdes sur l'épiderme ( « Retinoic acid receptor gamma mediates topical retinoid efficacy and irritation in animal models», Chen S, Ostrowski J, Whiting G, Roalsvig T, Hammer L, Currier SJ, Honeyman J, Kwasniewski B, Yu KL, Sterzycki R, et al. J Invest Dermatol. 1995;104 (5): 779-83).

Les récepteurs RAR gamma sont donc la seule cible dans le traitement de pathologies au niveau de l'épiderme comme par exemple pour l'acné ou le psoriasis ou toute autre pathologie cutanée traitée par les rétinoïdes.

D'autre part, certains effets secondaires propres à RAR alpha ou RAR beta peuvent être évités si on utilise des composés ayant une action sélective sur RAR gamma.

De façon surprenante, il a maintenant été montré que les composés selon l'invention présentent une activité agoniste sélective pour le sous-type gamma de la famille des récepteurs RAR extrêmement intéressante.

Les composés selon l'invention, agonistes sélectifs du sous-type RAR gamma, permettent ainsi de prévenir et/ou traiter diverses pathologies ou désordres dermatologiques, tout en diminuant les effets secondaires habituellement dus à l'action des actifs sur les sous-types RAR alpha et beta.

La présente invention a donc pour premier objet des composés qui peuvent être représentés par la formule générale suivante : 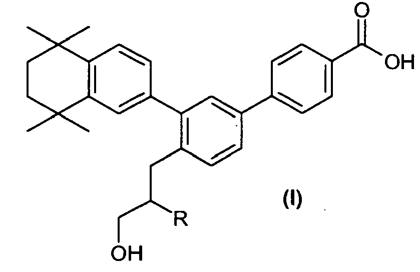 dans laquelle R représente un hydrogène ou un radical hydroxyl, ainsi que les sels des composés de formule (I).

Par sel pharmaceutiquement acceptable, on entend notamment un sel de métal alcalin, ou un sel alcalino-terreux, ou un sel d'amine organique.

Selon une forme de réalisation préférée, les composés de formule (I) sont choisis parmi l'acide 4'-(3-hydroxy-propyl)-3'-(5,5,8,8-tetramethyl-5,6,7,8-tetrahydronaphtalen-2-yl)-biphenyl-4-carboxylique, et l'acide 4'-(2,3-dihydroxy-propyl)-3'-(5,5,8,8-tetramethyl-5,6,7,8-tetrahydro-naphtalen-2-yl)-biphenyl-4-carboxylique.

L'invention vise également l'utilisation d'au moins un composé de formule (I) pour la préparation d'une composition pharmaceutique ou cosmétique destinée à prévenir et/ou traiter des pathologies pour lesquelles une activité agoniste sélective pour le sous-type gamma de la famille des récepteurs RAR est désirée.

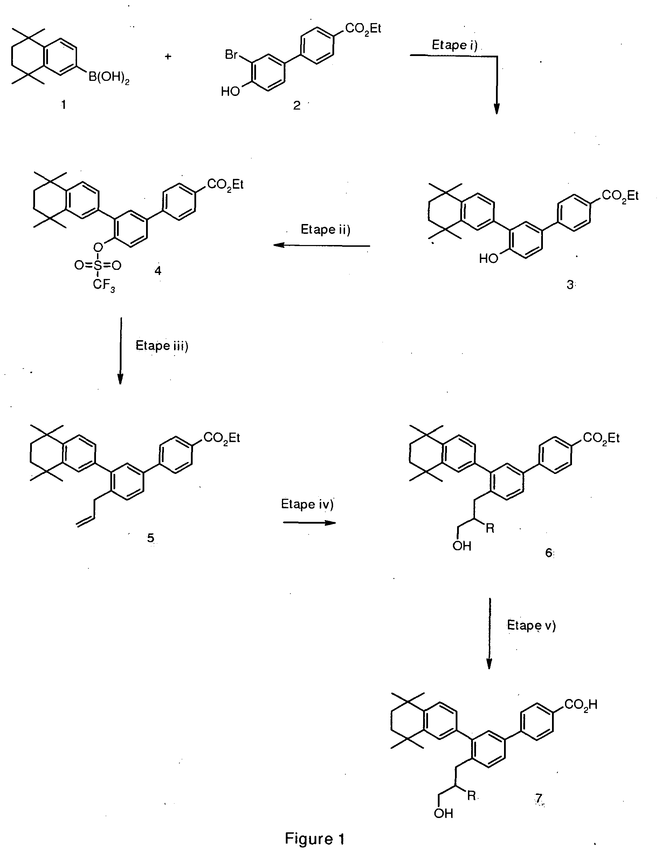

Une voie de synthèse générale pour préparer les composés de formule (I) est représentée dans le schéma selon la figure 1.

Les matières premières et/ou les réactifs utilisés sont disponibles commercialement et/ou peuvent être préparés selon des méthodes connues de la littérature.

Selon un autre aspect, la présente invention concerne également un procédé de préparation des composés de formule (I) précédemment décrits comprenant les étapes suivantes :
i) réaction de couplage, de préférence de type réaction de Suzuki, entre le composé de formule **1** préparé par exemple comme décrit dans la demande de brevet WO 99/10308 : 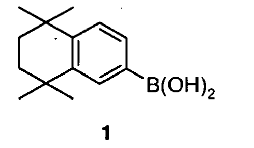 et le composé de formule **2** préparé par exemple comme décrit dans la demande de brevet WO 99/10308: 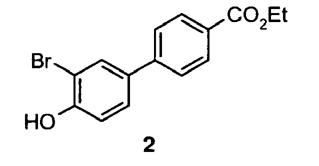 pour conduire au composé de formule **3**, 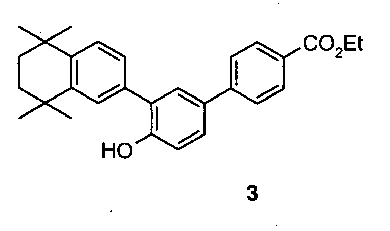
ii) réaction de la fonction phénol du composé de formule **3** avec l'anhydride triflique ((CF₃CO)₂O), selon des méthodes connues dans la littérature (voir par exemple Kotsuki H. & coll., Synthesis, 1990, (12), 1145-1147 ou Prince P. & coll., Synlett, 1991, (6), 405-406) pour conduire au composé de formule **4**, 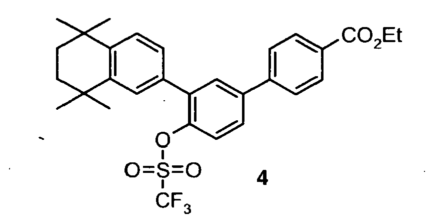
iii) réaction de type couplage de Stille du composé de formule **4** avec de l'allyle tri-*n*-butyle étain (CH₂=CH-CH₂-SnBu₃) selon des méthodes connues dans la littérature (voir par exemple Tilley J.W. & coll., J.Org.Chem. 1990, 55(3), 906 ou Saa J.M. & coll., J.Org.Chem. 1992, 57(2), 678-685) pour conduire au composé de formule **5,** 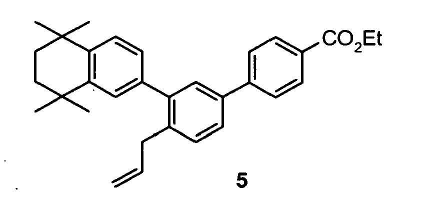
iv) hydratation de la double liaison allylique du composé de formule **5**, par exemple par une réaction classique d'hydroboration suivie d'une oxydation, selon des méthodes connues dans la littérature (voir par exemple : Liotta, R., Brown, H. C. J. Org. Chem. 1977, 42, 2836 ou Luo, F. T., Negishi, E. J. Org. Chem. 1983, 48, 5144) ou di-hydroxylation de la double liaison allylique du composé de formule 5 selon des méthodes connues dans la littérature (voir par exemple : Corey, E. J& coll. Tetrahedron Lett 1984, 25 (44), 5013, Sharpless, K. B. & coll. J. Am. Chem. Soc. 1976, 98 (7), 1986) pour conduire aux composés de formule **6,** avec respectivement R = H et R = OH : 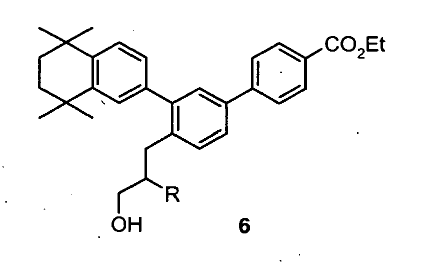
v) saponification de la fonction ester du composé de formule 6 pour conduire au composé de formule (I) (composé 7 dans la figure 1) dans laquelle R représente un hydrogène ou un radical hydroxyle.

L'étape i) peut par exemple être réalisée en présence de carbonate de potassium, de Tetrakis(triphénylphosphine)palladium dans une solution de toluène.

L'étape ii) peut par exemple être réalisée en présence d'anhydride trifluorométhanesulfonique et d'une base comme la triéthylamine dans un solvant aprotique comme le dichlorométhane.

L'étape iii) peut par exemple être réalisée en présence d'un catalyseur au palladium comme par exemple le chlorure de bis(triphenylphosphine) palladium (PdCl₂(PPh₃)₂) ou encore le tris(dibenzylidèneactone) dipalladium (Pd₂(dba)₃) dans un solvant polaire comme par exemple le diméthylformamide.

L'étape iv) peut par exemple être réalisée en présence de tetraoxyde d'osmium catalytique et d'un oxydant comme par exemple le N-oxyde de N-méthyl morpholine . Comme indiqué ci-dessus, dans ce cas la réaction conduit au composé 6 dans lequel R représente le radical hydroxyle.

Alternativement, l'étape iv) peut également être réalisée en présence d'un borane comme par exemple le 9-borabicyclo [3.3.1] nonane (9-BBN) suivi d'une oxydation utilisant par exemple de l'eau oxygénée. Dans ce cas, la réaction conduit au composé 6 dans lequel R représente l'hydrogène.

L'étape v) peut par exemple être réalisée en présence de d'hydroxyde de sodium et de THF.

La présente invention a également pour objet les composés de formule (I) tels que décrits ci-dessus à titre de médicament.

Selon un autre aspect, l'invention a pour objet une composition pharmaceutique ou cosmétique caractérisée en ce qu'elle comprend dans un véhicule pharmaceutiquement ou cosmétiquement acceptable au moins un composé de formule (I).

Par "véhicule pharmaceutiquement ou cosmétiquement acceptable", on entend un véhicule adapté pour une utilisation en contact avec des cellules d'humains et d'animaux, sans toxicité, irritation, réponse allergique indue et similaires, et proportionné à un rapport avantage/risque raisonnable.

L'administration peut être effectuée par voie topique, entérale ou orale, parentérale ou oculaire. Parmi ces voies d'administration, la voie topique est particulièrement préférée.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

Les composés sont utilisés par voie topique à une concentration généralement comprise entre 0,001 % et 3% en poids, par rapport au poids total de la composition.

Pour une application cosmétique, la composition est de préférence sous forme d'une crème, d'un lait, d'une lotion, d'un gel, de microsphères ou nanosphères ou vésicules lipidiques ou polymériques, d'un savon, d'un shampooing.

Par voie entérale ou orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,01 mg/kg à 30 mg/kg de poids corporel, en 1 à 3 prises.

Les composés de l'invention sont utiles, seuls ou en mélange, pour la préparation d'une composition pharmaceutique destinée au traitement et/ou à la prévention de pathologies liées à une déficience de l'activation du récepteur RAR gamma.

L'invention concerne également une méthode de traitement thérapeutique ou cosmétique, comprenant l'administration d'une composition pharmaceutique ou cosmétique comprenant au moins un composé de formule (I), ledit composé exerçant une activité agoniste sélective du récepteur RAR gamma.

La composition pharmaceutique peut être plus particulièrement destinée à traiter une pathologie pour le traitement de laquelle une activité agoniste sélective du récepteur RAR gamma est désirée, plus particulièrement au niveau des tissus épithéliaux, de la peau et des os.

La composition est notamment utile pour le traitement d'une pathologie liée aux désordres de la différenciation et/ou de la prolifération cellulaire, en particulier dans le domaine de la dermatologie.

Plus particulièrement, elle est utile pour le traitement d'une pathologie liée à un désordre de la kératinisation.

Le traitement de l'acné est ainsi envisagé, notamment les acnés vulgaires, comédoniennes, polymorphes, les acnés nodulokystiques, conglobata, les acnés séniles, les acnés secondaires tels que l'acné solaire, médicamenteuse, professionnelle.

La composition pharmaceutique comprenant un composé de formule (I) est également utile pour traiter d'autres affections dermatologiques liées à un trouble de la kératinisation avec une composante inflammatoire et/ou immunoallergique et, notamment, toutes les formes de psoriasis, qu'il soit cutané, muqueux ou unguéal.

Les composés selon l'invention sont également utiles dans une composition cosmétique, pour lutter contre le vieillissement cutané, qu'il soit par exemple photoinduit ou chronologique, ou encore pour le traitement des peaux à tendance acnéique ou pour lutter contre l'aspect gras de la peau ou des cheveux.

La composition pharmaceutique ou cosmétique peut permettre en outre la régulation des pigmentations de la peau et le traitement des kératoses actiniques.

Dans toutes les applications envisagées, ledit composé peut être associé à un autre agent thérapeutique utile dans le traitement d'une pathologie liée aux désordres de la différenciation ou de la prolifération cellulaire.

Comme agents thérapeutiques utilisables dans les compositions selon l'invention, on peut citer les agents modulant la différenciation et/ou la prolifération et/ou la pigmentation cutanée tels que l'acide rétinoïque et ses isomères, le rétinol et ses esters, le rétinal, les rétinoïdes, les estrogènes, les antibactériens, les antibiotiques, les antiparasitaires, les antifongiques, les agents anti-inflammatoires stéroïdiens ou non-stéroïdiens, les agents anesthésiques, les agents antiprurigineux, les agents antiviraux, les agents kératolytiques, les agents anti-radicaux libres, les anti-séborrhéiques, les antipelliculaires, les anti-acnéiques, les agents pour lutter contre la chute des cheveux, la vitamine C et ses dérivés sous réserve, comme cela est indiqué précédemment, que les actifs soient sous forme solubilisée dans la composition selon l'invention.

Les exemples suivants illustrent l'invention, sans toutefois la limiter. Les produits de départs utilisés sont des produits connus ou préparés selon des modes opératoires connus.

Dans les exemples ci-après, les échantillons ont été analysés par RMN ¹H et RMN ¹³C, HPLC/MS.

### Exemple 1 : Synthèse de l'acide 4'-(3-hydroxy-propyl)-3'-(5,5,8,8-tetramethyl-5,6,7,8-tetrahydro-naphtalen-2-yl)-biphenyl-4-carboxylique

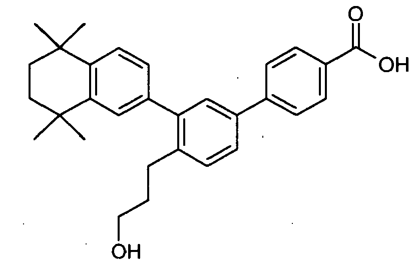

### a) 4'-Hydroxy-3'-(5,5,8,8-tetramethyl-5,6,7,8-tetrahydro-naphthalen-2-yl)-biphenyl-4-carboxylate d'éthyle:

Dans un tricol, 10 g (31 mmol) de 3'-bromo-4'-hydroxy-biphenyl-4-carboxylate d'éthyle (préparé selon EP952974), 8,7 g (37 mmol) d'acide 6-(1,1,4,4-tetraméthyl-1,2,3,4-tetrahydronaphtalène) boronique (préparé selon EP952974), et 34 mL (74,6 mmol) d'une solution aqueuse 2M de carbonate de potassium sont placés dans 200 mL de toluène, puis 1,8 g (1,55 mmol) de tetrakis (triphénylphosphine) palladium sont additionnés sous azote. Le mélange réactionnel est chauffé pendant 24 h à 110 °C. Après refroidissement, la réaction est arrêtée par addition de 200 mL d'eau puis extraite à l'acétate d'éthyle. Les phases organiques sont rassemblées, lavées par une solution de chlorure de sodium saturée et séchées sur sulfate de magnésium. Les solvants sont évaporés puis le résidu est purifié par chromatographie sur gel de silice (Heptane/Acétate d'éthyle 80/20).

8 g de 4'-hydroxy-3'-(5,5,8,8-tetramethyl-5,6,7,8-tetrahydro-naphthalen-2-yl)-biphenyl-4-carboxylate d'éthyle sont obtenus sous la forme d'un solide blanc (rendement = 60 %).

### b) 3'-(5,5,8,8-Tetramethyl-5,6,7,8-tetrahydro-naphthalen-2-yl)-4'-trifluoro methanesulfonyloxy-biphenyl-4-carboxylate d'éthyle:

Dans un tricol sous azote, 8,8 mL (63 mmol) de triéthylamine sont ajoutés goutte à goutte sur 18 g (42 mmol) de 4'-hydroxy-3'-(5,5,8,8-tetramethyl-5,6,7,8-tetrahydro-naphthalen-2-yl)-biphenyl-4-carboxylate d'éthyle en solution dans 250 mL de dichlorométhane. Le milieu réactionnel est refroidi vers 0°C puis 8,48 mL (50 mmol) d'anhydride trifluorométhanesulfonique sont additionnés goutte à goutte. Le milieu réactionnel est agité pendant 3h à 0°C, puis il est hydrolysé, lavé avec une solution de bicarbonate de soude et séché sur sulfate de magnésium. Après évaporation des solvants, le produit isolé est purifié par chromatographie sur gel de silice (Heptane/ Acétate d'éthyle = 95/5).

24g de 3'-(5,5,8,8-tetramethyl-5,6,7,8-tetrahydro-naphthalen-2-yl)-4'-trifluoro methanesulfonyloxy-biphenyl-4-carboxylate d'éthyle sont obtenus sous forme d'une poudre blanche (rendement = 100%).

### c) 4'-Allyl-3'-(5,5,8,8-tetramethyl-5,6,7,8-tetrahydro-naphthalen-2-yl)-biphenyl -4-carboxylate d'ethyle:

Dans un tricol sous azote, 6 g (10,7 mmol) de 3'-(5,5,8,8-tetramethyl-5,6,7,8-tetrahydro-naphthalen-2-yl)-4'-trifluoromethanesulfonyloxy-biphenyl-4-carboxylate d'éthyle, 0,9 g (21 mmol) de chlorure de lithium, 1,12 g (1,6 mmol) de dichlorure de bis(triphenylphosphine) palladium, 30 mL de dimethylformamide et 3,98 mL (12,8 mmol) d'allyle tri-n-butyle étain sont introduits dans cet ordre.

Le mélange réactionnel est agité pendant 12 h à 100°C. La réaction est hydrolysée avec une solution d'acide chlorhydrique 1 N puis extraite avec de l'acétate d'éthyle. Les phases organiques sont rassemblées, lavées par une solution aqueuse de chlorure de sodium saturée, et séchées sur sulfate de magnésium. Les solvants sont évaporés. Le brut obtenu sous forme d'huile est purifié par chromatographie sur gel de silice (Heptane/ Acétate d'éthyle : = 95/5).

6,1 g de 4'-allyl-3'-(5,5,8,8-tetramethyl-5,6,7,8-tetrahydro-naphthalen-2-yl)-biphenyl -4-carboxylate d'éthyle sont obtenus sous la forme d'une huile jaune claire (rendement = 100 %).

### d) 4'-(3-Hydroxy-propyl)-3'-(5,5,8,8-tetramethyl-5,6,7,8-tetrahydronaphthalen-2-yl)-biphenyl-4-carboxylate d'ethyle :

Dans un tricol sous azote, 0,5 g (1,1 mmol) de 4'-allyl-3'-(5,5,8,8-tetramethyl-5,6,7,8-tetrahydro-naphthalen-2-yl)-biphenyl-4-carboxylate d'éthyle sont introduits dans 20 mL de tétrahydrofuranne. Cette solution est refroidie à 0°C, puis 6,6 mL (3,3 mmol) d'une solution de 9-borabicyclo[3.3.1]nonane 0,5 M dans le tétrahydrofuranne est additionnée goutte à goutte. Après complète addition, le milieu réactionnel est agité jusqu'à revenir à température ambiante. Il est agité encore une heure à cette température, puis refroidi de nouveau vers 0°C. 3,4 mL (3,4 mmol) d'une solution 1 M de soude sont additionnés lentement au milieu réactionnel, puis 2,8 mL (27,6 mmol) d'une solution d'eau oxygénée à 30% sont additionnés goutte à goutte. Après complète addition, le milieu réactionnel est agité en laissant remonter la température à l'ambiant. Le milieu est agité 12h à cette température, puis il est hydrolysé avec une solution de chlorure d'ammonium. La phase aqueuse est extraite à l'acétate d'éthyle et les phases organiques réunies sont lavées à l'eau, puis séchées sur sulfate de magnésium. Les solvants sont évaporés. Le brut obtenu est purifié par chromatographie sur gel de silice (Heptane/Acétate d'éthyle : = 95/5)

300 mg de 4'-(3-hydroxy-propyl)-3'-(5,5,8,8-tetramethyl-5,6,7,8-tetrahydronaphthalen-2-yl)-biphenyl-4-carboxylate d'éthyle sont obtenus sous la forme d'une huile incolore (rendement = 58 %).

### e) Acide 4'-(3-hydroxy-propyl)-3'-(5,5_{,}8,8-tetramethyl-5,6,7,8-tetrahydronaphtalen-2-yl)-biphenyl-4-carboxyliaue:

1 mL (1 mmol) d'une solution aqueuse d'hydroxyde de sodium (1 M) est ajouté à une solution de 300 mg (0,64 mmol) de 4'-(3-hydroxy-propyl)-3'-(5,5,8,8-tetramethyl-5,6,7,8-tetrahydro-naphthalen-2-yl)-biphenyl-4-carboxylate d'éthyle dans 15 mL de tétrahydrofuranne et 1 mL d'éthanol. Le mélange réactionnel est agité pendant 14 h en chauffant à 50 °C. Après être revenu à température ambiante, 2 mL (2 mmol) d'une solution d'acide chlorhydrique (1 N) sont additionnés. Le milieu est extrait à l'acétate d'éthyle. La phase organique est lavée à l'eau, puis séchée sur sulfate de magnésium. Après évaporation des solvants, le produit obtenu est purifié par chromatographie sur gel de silice (Heptane/ Acétate d'éthyle : = 3/2).

230 mg d'acide 4'-(3-hydroxy-propyl)-3'-(5,5,8,8-tetramethyl-5,6,7,8-tetrahydro-naphthalen-2-yl)-biphenyl-4-carboxylique sont obtenus (solide blanc, P.F.=203°C, rendement = 90 %).

### Exemple 2 : Synthèse de l'acide 4'-(2,3-Dihydroxy-propyl)-3'-(5,5,8,8-tetramethyl-5,6,7,8-tetrahydro-naphtalen-2-yl)-biphenyl-4-carboxylique

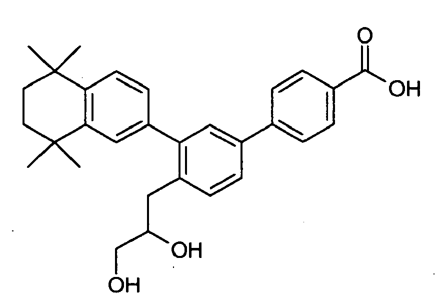

### a) 4'-(2,3-Dihydroxy-propyl)-3'-(5,5,8,8-tetramethyl-5,6,7,8-tetrahydronaphthalen-2-yl)-biphenyl-4-carboxylate d'éthyle:

Dans un tricol sous azote, 2,84 g (6,3 mmol) de 4'-allyl-3'-(5,5,8,8-tetramethyl-5,6,7,8-tetrahydro-naphthalen-2-yl)-biphenyl-4-carboxylate d'éthyle (préparé selon exemple 1, étape c) sont introduits dans 15 mL de dichlorométhane, puis 1,0 g (7,5 mmol) de N-oxyde de N-methyl morpholine est ajouté.

2,27 mL (0,18 mmol) d'une solution de tetraoxyde d'osmium à 2,5% en poids dans l'isopropanol sont additionnés à température ambiante. Le milieu réactionnel devient jaune foncé. Il est agité pendant 3h à température ambiante, puis il est hydrolysé et extrait au dichlorométhane. Les phases organiques réunies sont lavées au thiosulfate de sodium puis séchées sur sulfate de magnésium. Après évaporation des solvants, le produit obtenu est purifié par chromatographie sur gel de silice (Heptane/ Acétate d'éthyle = 1/1).

1,92 g de 4'-(2,3-dihydroxy-propyl)-3'-(5,5,8,8-tetramethyl-5,6,7,8-tetrahydronaphthalen-2-yl)-biphenyl-4-carboxylate d' éthyle sont obtenus sous forme d'une huile beige (rendement = 63 %).

### b) acide 4'-(2,3-dihydroxy-propyl)-3'-(5,5,8,8-tetramethyl-5,6,7,8-tetrahydronaphtalen-2-yl)-biphenyl-4-carboxylique:

1 mL (1 mmol) d'une solution aqueuse d'hydroxyde de sodium (1 M) est ajouté à une solution de 200 mg (0,41 mmol) de 4'-(2,3-dihydroxy-propyl)-3'-(5,5,8,8-tetramethyl-5,6,7,8-tetrahydro-naphthalen-2-yl)-biphenyl-4-carboxylate d' éthyle dans 5 mL de tétrahydrofuranne et 2 mL d'éthanol. Le mélange réactionnel est agité pendant 48 h en chauffant à 50 °C. Après être revenu à température ambiante, 2 mL (2 mmol) d'une solution d'acide chlorhydrique (1 N) sont additionnés. Le milieu est extrait à l'acétate d'éthyle. La phase organique est lavée à l'eau, puis séchée sur sulfate de magnésium. Après évaporation des solvants, le produit obtenu est purifié par cristallisation dans un mélange heptane/ Ether diéthylique.

180 mg d'acide 4'-(2,3-dihydroxy-propyl)-3'-(5,5,8,8-tetramethyl-5,6,7,8-tetrahydro-naphtalen-2-yl)-biphenyl-4-carboxylique sont obtenus (solide beige, P.F.=145°C, rendement = 96 %).

### Exemple 3 : Test de transactivation

### a) Principe du test :

L'activation des récepteurs par un agoniste (activateur) dans des cellules HeLa conduit à l'expression d'un gène reporter, la luciférase, qui, en présence d'un substrat génère de la lumière. On peut donc mesurer l'activation des récepteurs en quantifiant la luminescence produite après incubation des cellules en présence d'un antagoniste de référence. Les produits activateurs déplacent l'antagoniste de son site permettant ainsi l'activation du récepteur. La mesure de l'activité se fait par la quantification de l'augmentation de la lumière produite. Cette mesure permet de déterminer l'activité activatrice du composé utile dans l'invention.

Dans cette étude est déterminée une constante qui représente l'affinité de la molécule pour le récepteur. Cette valeur pouvant fluctuer selon l'activité basale et l'expression du récepteur, on la désigne Kd apparent (KdApp).

Pour déterminer cette constante, des « courbes croisées » du produit à tester (acide 4'-(3-hydroxy-propyl)-3'-(5,5,8,8-tetramethyl-5,6,7,8-tetrahydro-naphthalen-2-yl)-biphenyl-4-carboxylique et acide 4'-(2,3-dihydroxy-propyl)-3'-(5,5,8,8-tetramethyl-5,6,7,8-tetrahydro-naphtalen-2-yl)-biphenyl-4-carboxylique), contre un antagoniste de référence autrement nommé ligand de référence, l'acide 4-(5,5-dimethyl-8-p-tolyl-5,6-dihydro-naphthalen-2-ylethynyl)-benzoïque sont réalisées. Le produit à tester est utilisé à 10 concentrations et l'antagoniste de référence à 7 concentrations. Dans chaque puit (d'une plaque à 96 puits), les cellules sont en contact d'une concentration du produit à tester et d'une concentration de l'antagoniste de référence.

Des mesures sont également réalisées pour les témoins agoniste total autrement nommé témoin 100% (l'acide 4-[2-(5,5,8,8 tetramethyl-5,6,7,8 tetrahydronaphtalene-2-yl)propenyl]-benzoique) et agoniste inverse autrement nommé témoin 0%, (l'acide 4-{(E)-3-[4-(4-tert-butyl-phenyl)-5,5,8,8-tetramethyl-5,6,7,8-tetrahydro-naphthalen-2-yl]-3-oxo-propenyl)-benzoïque).

Ces courbes croisées permettent de déterminer les AC50 (concentration à laquelle on observe 50% d'activation du récepteur) du ligand de référence à différentes concentrations de produit à tester. Ces AC50 sont utilisées pour calculer la régression de Schild en traçant une droite répondant à l'équation de Schild (« quantitation in receptor pharmacology » Terry P.Kenakin, Receptors and Channels, 2001,7, 371-385).

Dans le cas d'un agoniste, l'AC50 est calculée en traçant la courbe du produit à la concentration du ligand de référence donnant 80% d'activation. Le pourcentage d'activation qui correspond au niveau maximum d'activité obtenue est également mesuré.

### b) Matériels et Méthode :

Les lignées cellulaires HeLa utilisées sont des transfectants stables contenant les plasmides ERE-βGlob-Luc-SV-Neo (gène reporter) et RAR (α, β, γ) ER-DBD-puro. Ces cellules sont ensemencées en plaques 96 puits à raison de 10 000 cellules par puit dans 100µl de milieu DMEM sans rouge de phénol et supplémenté par 10% de sérum de veau délipidé. Les plaques sont ensuite incubées à 37°C, 7% CO₂ pendant 4 heures.

Les différentes dilutions du produit à tester, du ligand de référence (l'acide 4-(5,5-dimethyl-8-p-tolyl-5,6-dihydro-naphthalen-2-ylethynyl)-benzoique), du témoin 100% (l'acide 4-[2-(5,5,8,8 tetramethyl-5,6,7,8 tetrahydronaphtalene-2-yl)propenyl]-benzoïque 100 nM) et du témoin 0% (l'acide 4-{(E)3-[4-(4-tert-butyl-phenyl)-5,5,8,8-tetramethyl-5,6,7,8-tetrahydro-naphthalen-2-yl]-3-oxo-propenyl}-benzoïque 500 nM) sont rajoutées à raison de 5 µl par puits. Les plaques sont ensuite incubées 18 heures à 37°C, 7% CO₂.

Le milieu de culture est éliminé par retournement et 100 µl d'un mélange 1:1 PBS (solution tampon de phosphate)/Luciférine est ajouté à chaque puit. Après 5 minutes, les plaques sont lues par le lecteur de luminescence.

### c) Résultats :

Les valeurs des constantes Kd apparent sont indiquées dans le tableau ci-après. Ces valeurs sont comparées à celles des composés de la demande de brevet WO 99/10308 présentant les meilleures activités

| | RAR alpha Kdapp (nM) | RAR béta Kdapp (nM) | RAR gamma Kdapp (nM) |
|---|---|---|---|
| Composé de l'exemple 1 | 15 | 2 | 0.1 |
| Composé de l'exemple 2 | 60 | 15 | 0.5 |
| **Composés de** WO 99/10308 **:** | | | |
| Acide 3"-methyl-2'-(5,5,8,8-tetramethyl-5,6,7,8-tetrahydro-naphtalén-2-yl)-[1,1' ;4',1"]terphenyl-4"-carboxylique (Exemple41) | 2 | 1 | 0,25 |
| Acide 3"-hydroxy-2'-(5,5,8,8-tetramethyl-5,6,7,8-tetrahydro-naphtalen-2-yl)-[1,1';4',1"]terphenyl-4"-carboxylique (Exemple46) | 2 | 1 | 0,25 |
| Acide 2"-methoxy-2'-(5,5,8,8-tetramethyl-5,6,7,8-tetrahydro-naphtalen-2-yl)-[1,1';4',1"]terphenyl-4"-carboxylique(Exemple44) | 4 | 2 | 0,5 |
| Acide 2"-hydroxy-2'-(5,5,8,8-tetramethyl-5,6,7,8-tetrahydro-naphtalen-2-yl)-[1,1';4',1"]terphenyl-4"-carboxylique (Exemple42) | 8 | 4 | 1 |
| Acide 6-[2-(5,5,8,8-tetramethyl-5,6,7,8-tetrahydro-naphtalen-2-yl)-biphenyl-4-yl]-nicotinique (Exemple47) | 4 | 1 | 1 |
| Acide 2'-(5,5,8,8-tetramethyl-5,6,7,8-tetrahydro-naphtalen-2-yl)-[1,1' ;4',1"] terphenyl-4"-carboxylique (Exemple14) | 2 | 1 | 1 |

Les résultats obtenus avec l'acide 4'-(3-hydroxy-propyl)-3'-(5,5,8,8-tetramethyl-5,6,7,8-tetrahydro-naphthalen-2-yl)-biphenyl-4-carboxylique, et l'acide 4'-(2,3-dihydroxy-propyl)-3'-(5,5,8,8-tetramethyl-5,6,7,8-tetrahydro-naphtalen-2-yl)-biphenyl-4-carboxylique montrent bien une meilleure sélectivité de ces composés pour le sous-type de récepteur RARgamma en comparaison avec les deux autres sous-types RARalpha et RARbéta. Ils montrent également une meilleure activité et une meilleure sélectivité en comparaison avec les composés RAR les plus actifs décrits dans la demande de brevet WO 99/10308.

Ces composés sont donc des agonistes ou activateurs sélectifs du récepteur RARgamma.

### Exemple 4 : Exemples de formulations

Dans cet exemple, on a illustré diverses formulations concrètes à base des composés selon l'invention.

### A- VOIE ORALE

### (a) Comprimé de 0,2 g

- Acide 4'-(3-Hydroxy-propyl)-3'-(5,5,8,8-tetramethyl-5,6,7,8-tetrahydronaphthalen-2-y)-biphenyl-4-carboxylique 0,001 g
- Amidon 0,114 g
- Phosphate bicalcique 0,020 g
- Silice 0,020 g
- Lactose 0,030 g
- Talc 0,010 g
- Stéarate de magnésium 0,005 g

### (b) Suspension buvable en ampoules de 5 ml

- Acide 4'-(3-Hydroxy-propyl)-3'-(5,5,8,8-tetramethyl-5,6,7,8-tetrahydronaphthalen-2-yl)-biphenyl-4-carboxylique 0,001 g
- Glycérine 0,500 g
- Sorbitol à 70% 0,500 g
- Saccharinate de sodium 0,010 g
- Parahydroxybenzoate de méthyle 0,040 g
- Arome qs
- Eau purifiée qsp 5 ml

### B- VOIE PARENTERALE

### (a) Composition

- Acide 4'-(2,3-Dihydroxy-propyl)-3'-(5,5,8,8-tetramethyl-5,6,7,8-tetrahydronaphtalen-2-yl)-biphenyl-4-carboxylique 0,05 %
- Polyéthylène glycol 20%
- Solution de NaCl à 0.9% qs 100

### (b) Composition de cyclodextrine injectable

- Acide 4'-(2,3-Dihydroxy-propyl)-3'-(5,5,8,8-tetramethyl-5,6,7,8-tetrahydronaphtalen-2-yl)-biphenyl-4-carboxylique 0,1 mg
β- cyclodextrine 0,10 g
Eau pour injectable qsp.10,00 g

### C- VOIE TOPIQUE

### (a) Onguent

- Acide 4'-(3-Hydroxy-propyl)-3'-(5,5,8,8-tetramethyl-5,6,7,8-tetrahydronaphthalen-2-yl)-biphenyl-4-carboxylique 0,300 g
- Vaseline blanche codex qsp 100 g

### (b) Crème Eau-dans-Huile non ionique

- Acide 4'-(2,3-Dihydroxy-propyl)-3'-(5,5,8,8-tetramethyl-5,6,7,8-tetrahydronaphtalen-2-yl)-biphenyl-4-carboxylique 0,100 g
- Mélange d'alcools de lanoline émulsifs, de cires et d'huiles ("Eucerine anhydre" vendu par BDF) 39,900 g
- Parahydroxybenzoate de méthyle 0,075 g
- Parahydroxybenzoate de propyle 0,075 g
- Eau déminéralisée stérile qsp 100 g

### (c) Lotion

- Acide 4'-(3-Hydroxy-propyl)-3'-(5,5,8,8-tetramethyl-5,6,7,8-tetrahydronaphthalen-2-yl)-biphenyl-4-carboxylique 0,100 g
- Polyéthylène glycol (PEG 400) 69,900 g
- Ethanol à 95% 30,000 g

## Revendications

1. Composés biphényliques de formule (I) 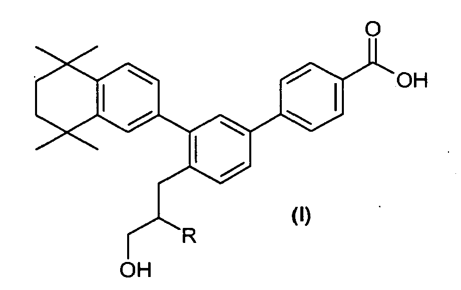 dans laquelle R représente un hydrogène ou un radical hydroxyl ainsi que les sels des composés de formule (I).

2. Composé selon la revendication 1, **caractérisé en ce qu'**il est choisi parmi l'un des composés suivants :
- l'acide 4'-(3-hydroxy-propyl)-3'-(5,5,8,8-tetramethyl-5,6,7,8-tetrahydro-naphthalen-2-yl)-biphenyl-4-carboxylique; et
- l'acide 4'-(2,3-dihydroxy-propyl)-3'-(5,5,8,8-tetramethyl-5,6,7,8-tetrahydronaphtaten-2-yl)-biphenyl-4-carboxylique.

3. Composé selon l'une quelconque des revendications précédentes à titre de médicament.

4. Composition pharmaceutique ou cosmétique **caractérisée en ce qu'**elle comprend dans un véhicule pharmaceutiquement ou cosmétiquement acceptable au moins un composé de formule (I) selon l'une des revendications 1 ou 2.

5. Composition pharmaceutique ou cosmétique selon la revendication 4, **caractérisée en ce qu'**elle est sous forme adaptée pour une administration par voie topique.

6. Composition selon la revendication 5, **caractérisée en ce que** la quantité de composé de formule (I) est comprise entre 0,001% et 3% en poids par rapport au poids total de la composition.

7. Utilisation d'un composé de formule (I) tel que défini dans l'une des revendications 1 ou 2 pour la préparation d'une composition pharmaceutique destinée à la prévention et/ou au traitement de pathologies liées à une déficience de l'activation du récepteur RAR gamma.

8. Utilisation selon la revendication 7, pour le traitement d'une pathologie liées aux désordres de la différenciation et/ou de la prolifération cellulaire.

9. Utilisation selon l'une des revendications 7 ou 8, pour le traitement d'une pathologie liée à un désordre de la kératinisation.

10. Utilisation selon l'une des revendications 7 à 9, pour le traitement de l'acné.

11. Utilisation selon l'une des revendications 7 à 9, pour le traitement du psoriasis.

12. Procédé de préparation d'un composé de formule (I) selon l'une des revendications 1 ou 2, comprenant les étapes suivantes :
i) la réaction de Suzuki entre le composé de formule 1 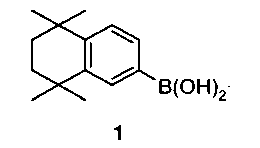 et le composé de formule 2 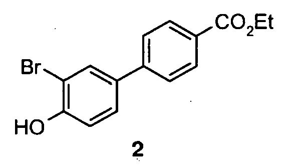 pour conduire au composé de formule 3, 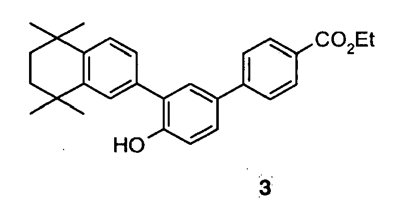
ii) la réaction du composé de formule 3 avec l'anhydride triflique pour conduire au composé de formule 4, 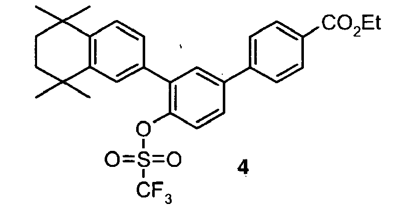
iii) la réaction du composé de formule 4 avec de l'allyle tri-n-butyle étain pour conduire au composé de formule 5, 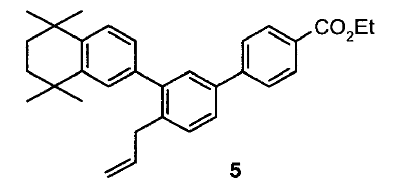
iv) l'hydratation ou la dihydroxylation de la double liaison allylique du composé de formule 5 pour conduire au composé de formule 6 dans laquelle R représente respectivement un hydrogène ou un radical hydroxyle. 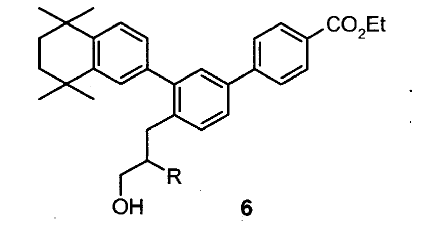
v) la saponification de la fonction ester du composé de formule 6.

## Claims

1. Biphenyl compounds of formula (I) 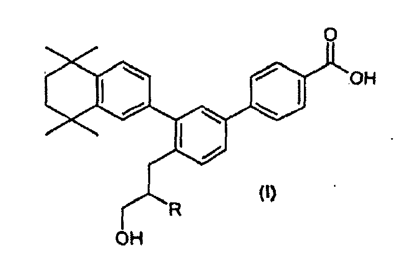 in which R represents a hydrogen or a hydroxyl radical, and also the salts of the compounds of formula (I).

2. Compound according to Claim 1, **characterized in that** it is chosen from one of the following compounds:
- 4'-(3-hydroxypropyl)-3'-(5,5,8,8-tetramethyl-5,6,7,8-tetrahydronaphthalen-2-yl)biphenyl-4-carboxylic acid; and
- 4'-(2,3-dihydroxypropyl)-3'-(5,5,8,8-tetramethyl-5,6,7,8-tetrahydronaphthalen-2-yl)biphenyl-4-carboxylic acid.

3. Compound according to either one of the preceding claims, as a medicament.

4. Pharmaceutical or cosmetic composition, **characterized in that** it comprises, in a pharmaceutically or cosmetically acceptable carrier, at least one compound of formula (I) according to either of Claims 1 and 2.

5. Pharmaceutical or cosmetic composition according to Claim 4, **characterized in that** it is in a form suitable for topical administration.

6. Composition according to Claim 5, **characterized in that** the amount of compound of formula (I) is between 0.001% and 3% by weight, relative to the total weight of the composition.

7. Use of a compound of formula (I) as defined in either of Claims 1 and 2, in the preparation of a pharmaceutical composition for use in the prevention and/or treatment of pathologies linked to a deficiency of the activation of the RAR gamma receptor.

8. Use according to Claim 7, in the treatment of a pathology linked to cell differentiation and/or proliferation disorders.

9. Use according to either of Claims 7 and 8, in the treatment of a pathology linked to a keratinization disorder.

10. Use according to one of Claims 7 to 9, in the treatment of acne.

11. Use according to one of Claims 7 to 9, in the treatment of psoriasis.

12. Process for preparing a compound of formula (I) according to either of Claims 1 and 2, comprising the following steps:
i) Suzuki reaction between the compound of formula 1 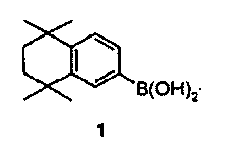 and the compound of formula 2 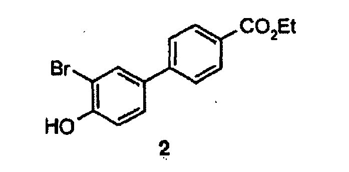 so as to produce the compound of formula 3 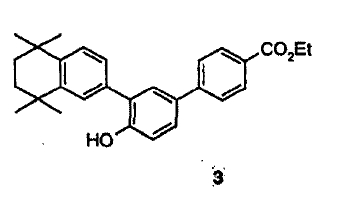
ii) reaction of the compound of formula **3** with triflic anhydride so as to produce the compound of formula **4** 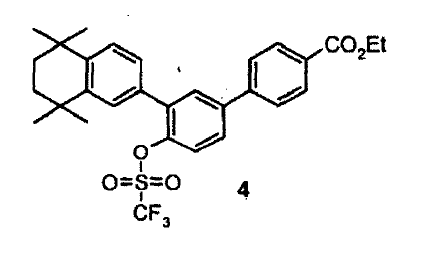
iii) reaction of the compound of formula **4** with allyl tri-*n*-butyl tin so as to produce the compound of formula **5** 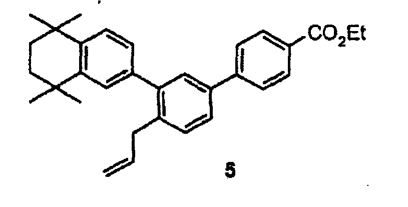
iv) hydration or dihydroxylation of the allylic double bond of the compound of formula 5 so as to produce the compound of formula **6** in which R represents, respectively, a hydrogen or a hydroxyl radical 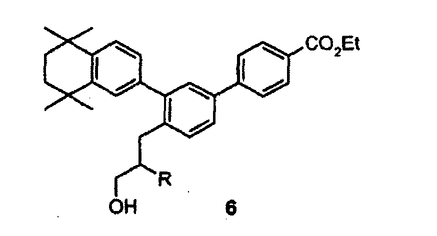
v) saponification of the ester function of the compound of formula **6**.

## Patentansprüche

1. Biphenylverbindungen der Formel (I): 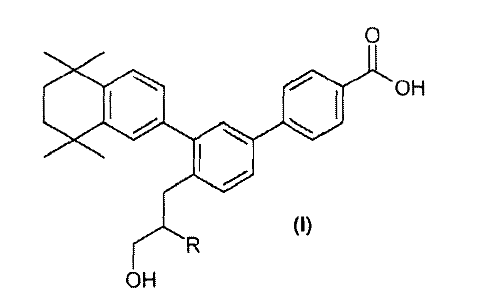 worin R Wasserstoff oder eine Hydroxygruppe bedeutet, sowie die Salze der Verbindungen der Formel (I).

2. Verbindung nach Anspruch 1, **dadurch gekennzeichnet, dass** sie unter den folgenden Verbindungen ausgewählt ist:
4'-(3-Hydroxy-propyl)-3'-(5,5,8,8-tetramethyl-5,6,7,8-tetrahydronaphthalin-2-yl)-biphenyl-4-carbonsäure; und
4'-(2,3-Dihydroxy-propyl)-3'-(5,5,8,8-tetramethyl-5,6,7,8-tetrahydronaphthalin-2-yl)-biphenyl-4-carbonsäure.

3. Verbindung nach einem der vorhergehenden Ansprüche als Arzneimittel.

4. Pharmazeutische oder kosmetische Zusammensetzung, **dadurch gekennzeichnet, dass** sie in einem pharmazeutisch oder kosmetisch akzeptablen Träger mindestens eine Verbindung der Formel (I) nach einem der Ansprüche 1 oder 2 enthält.

5. Pharmazeutische oder kosmetische Zusammensetzung nach Anspruch 4, **dadurch gekennzeichnet, dass** sie in einer Form vorliegt, die für eine Verabreichung auf topischem Weg geeignet ist.

6. Zusammensetzung nach Anspruch 5, **dadurch gekennzeichnet, dass** der Mengenanteil der Verbindung der Formel (I) im Bereich von 0,001 bis 3 Gew.-%, bezogen auf das Gesamtgewicht der Zusammensetzung, liegt.

7. Verwendung einer Verbindung der Formel (I), wie sie in einem der Ansprüche 1 oder 2 definiert ist, für die Herstellung einer pharmazeutischen Zusammensetzung, die für die Vorbeugung und/ oder Behandlung von Erkrankungen vorgesehen ist, die mit einer mangelnden Aktivierung des RAR-Rezeptors gamma zusammenhängen.

8. Verwendung nach Anspruch 7 für die Behandlung einer Erkrankung, die mit Störungen der zellulären Differenzierung und/ oder der Proliferation verbunden sind.

9. Verwendung nach Anspruch 7 oder 8 für die Behandlung einer Erkrankung, die mit einer Keratinisierungsstörung verbunden ist.

10. Verwendung nach einem der Ansprüche 7 bis 9 für die Behandlung von Akne.

11. Verwendung nach einem der Ansprüche 7 bis 9 für die Behandlung von Psoriasis.

12. Verfahren zur Herstellung einer Verbindung der Formel (I) nach einem der Ansprüche 1 oder 2, das die folgenden Schritte umfasst:
i) Suzuki-Reaktion zwischen der Verbindung der Formel 1 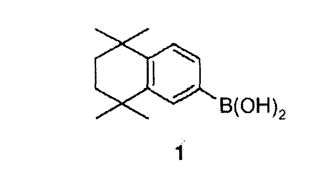 und der Verbindung der Formel 2 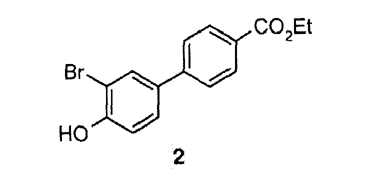 zur Bildung der Verbindung der Formel 3 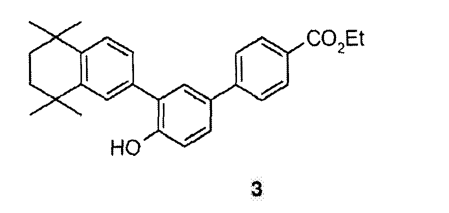
ii) Umsetzung der Verbindung der Formel 3 mit Trifluormethansulfonsäureanhydrid zur Bildung der Verbindung der Formel 4 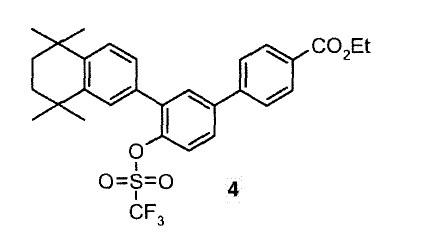
iii) Umsetzung der Verbindung der Formel 4 mit Allyl-tri-n-butylzinn zur Bildung der Verbindung der Formel 5 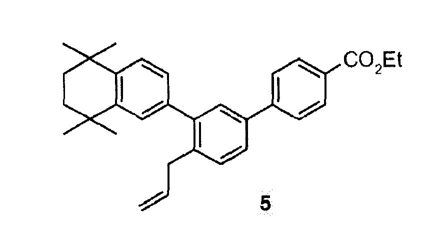
iv) Hydratisierung oder Dihydroxylierung der Allyldoppelbindung der Verbindung der Formel 5 zur Bildung der Verbindung der Formel 6, worin R Wasserstoff bzw. Hydroxy bedeutet 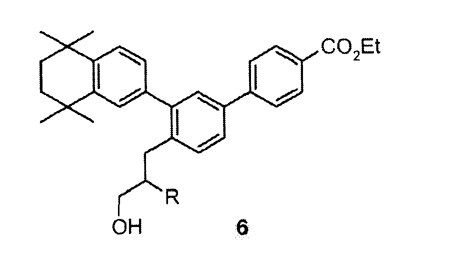
v) Verseifen der Esterfunktion der Verbindung der Formel 6.
